# EUROPEAN PATENT SPECIFICATION

(11) **EP 3 213 086 B1**
(45) Date of publication and mention of the grant of the patent: **08.01.2020**
(21) Application number: 14906310.9
(22) Date of filing: 18.11.2014
(51) Int. Cl.: G01N 33/72, A61K 8/22, A61K 8/41, A61K 8/36, A61K 8/44, A61Q 11/00, A61P 1/02

(54) **A KIT COMPRISING A COMPOSITION FOR DETECTING BLOOD**
KIT MIT EINE ZUSAMMENSETZUNG ZUM NACHWEIS VON BLUT
KIT COMPRENANT UN COMPOSITION POUR LA DÉTECTION DE SANG

(43) Date of publication of application: 06.09.2017
(73) Proprietor: Colgate-Palmolive Company, New York, NY 10022 (US)
(72) Inventor: XU, Shao Peng, Guangzhou Guangdong (CN); QIN, Xiong Fei, Zengcheng Guangdong (CN); YAN, Peng, Guangzhou Guangdong 510730 (CN)
(74) Representative: Wibbelmann, Jobst
(86) International application number: PCT/CN2014/091418
(87) International publication number: WO 2016/077996

(56) References cited:
- WO-A1-97/35030
- JP-A- 2002 181 815
- US-A- 4 038 485
- US-A- 4 587 220
- US-A- 5 217 874
- US-A1- 2003 170 905

## Description

### TECHNICAL FIELD

The present invention generally relates to kits for detecting blood, particularly occult blood in saliva. Also described is the use of a surfactant to reduce the time taken to detect blood.

### BACKGROUND

Bleeding from the gums after brushing is a common symptom of gingivitis, which can lead to more serious disorders if left untreated. Oral care compositions for the treatment of gum bleeding are commercially available. However, it is difficult for consumers to observe the effects of these compositions because they typically rely on their own subjective visual evaluation of the severity of the bleeding. In addition, in the early stages of gingivitis the amount of bleeding may be too small to be observed by eye.

Various methods of detecting bleeding have been proposed.

EP 0253548 discloses a method for the determination of pseudo-peroxidases, especially for the determination of occult blood in specimens of human material, which employs stable liquid chemical compositions containing chromogenic materials and a peroxide.

US 3,252,762 discloses blood testing compositions wherein one component of the test is encapsulated by means of an encapsulating material.

US 4,676,950 discloses a test composition for detecting occult blood which includes an alkaline water insoluble fraction of guaiac, an organic hydroperoxide containing a tertiary hydroperoxide group, and a buffer capable of maintaining the pH of material being tested within the range of about 4.5 to about 6.9, the purified guaiac, the hydroperoxide and the buffer being present in an amount effective to produce a color change in the presence of a hemoglobin containing material.

Although these methods are effective in detecting blood, their use for consumer applications is limited because the appearance of a visible color change is slow. There remains a need in the art for improved compositions and methods for detecting blood.

WO97/35030 A1 discloses a solution-based assay for determining the presence of a peroxidatively-active substance in a bodily fluid.
JP 2002 181815 A provides an assay method for measuring a saliva constituent by bringing a mouth-wash liquid discharged after washing the inside of a mouth into contact with an anti-saliva constituent anti body.
US 4038845 A concerns a test device, test composition, and method for determining the presence of a component in a sample. The device may comprise a carrier matrix incorporated with a reactant system which produces a detectable response upon contact with the sample component.
US 4587220 A discloses a composition, test means and device as well as method for determining peroxidatively active substances in a test sample. The composition, test means (and device) and the method are rendered resistant to the adverse effects of ascorbate which may be present in the sample by the inclusion in the composition of a metal chelate.
US 5217874 A discloses a test sheet for the determination of the presence of a substance having peroxidase-like activity in a throw-in-the-bowl fecal occult blood test.

### BRIEF SUMMARY

The present invention provides a kit comprsing:
(a) a composition for detecting blood on a solid object, which composition includes:
   (i) 3,3',5,5'-tetramethylbenzidine,
   (ii) cumene hydroperoxide, and
   (iii) a surfactant;
   wherein the composition is an aqueous solution; and wherein the surfactant is an anionic surfactant or a non-ionic surfactant, and
(b) an oral care composition.

It has surprisingly been found that the presence of a surfactant increases the rate of oxidation of 3,3',5,5'-tetramethylbenzidine by cumene hydroperoxide in solution in the presence of blood. This allows blood to be detected more rapidly.

The surfactant is preferably an anionic surfactant. Anionic surfactants have been found to produce the largest increase in reaction rate. The anionic surfactant may be an alkyl sulfate surfactant or an alkyl ether sulfate surfactant. The alkyl ether sulfate surfactant may be sodium lauryl ether sulfate. The alkyl sulfate surfactant may be ammonium lauryl sulfate.

Alternatively, the surfactant may be a non-ionic surfactant. Non-ionic surfactants have also been found to be effective in increasing the rate of reaction. The non-ionic surfactant may be a fatty acid amide. A preferred fatty acid amide is coconut mono ethanol amide.

The present invention provides a kit comprising the composition and an oral care composition. Such a kit allows a consumer to monitor the effect of the oral care composition on gum bleeding. Gum bleeding is a common symptom of gingivitis, and the oral care composition is therefore preferably an oral care composition for use in the treatment of gingivitis.

In another aspect, the present invention provides a method for detecting blood in a sample, the blood including a haemoglobin, which method comprises:
(a) contacting the sample with a composition as defined above wherein the oxidation of the 3,3',5,5'-tetramethylbenzidine by the cumene hydroperoxide is catalysed by the haemoglobin, and
(b) detecting the oxidation of the 3,3',5,5'-tetramethylbenzidine so as to detect the presence of blood.

It has been found that the surfactant increases the rate of reaction, thereby allowing the more rapid detection of the blood.

The sample may comprise saliva. The detection of blood in saliva is useful for allowing the diagnosis of diseases and disorders of the oral cavity, such as gingivitis

.The sample may be carried on a toothbrush. The analysis of a sample on a toothbrush allows the convenient detection of gum bleeding, because gum bleeding is most commonly observed after brushing.

The detection may be quantitative. Quantitative detection allows, for example, the progress of a therapy to be tracked over time and may provide some indication of the severity of bleeding which may be useful for diagnostic purposes.

The oxidation of 3,3',5,5'-tetramethylbenzidine results in a colour change. Step (b) may therefore comprise detecting the oxidation using a UV visible spectrometer or a colorimeter. Spectroscopic methods allow the precise measurement of the extent of oxidation, and therefore the extent of bleeding.

In a still further aspect, the present invention includes the use of a surfactant to reduce the time taken to detect blood using 3,3',5,5'-tetramethylbenzidine and cumene hydroperoxide in an aqueous solution, wherein the surfactant is an anionic surfactant or a non-ionic surfactant. It has surprisingly been found that anioic surfactants and non-ionic surfactants increase the rate of oxidation of 3,3',5,5'-tetramethylbenzidine in the presence of cumene hydroperoxide and blood. The anionic surfactant may be an alkyl sulfate surfactant or an alkyl ether sulfate surfactant. The non-ionic surfactant may be a fatty acid amide.

The blood may be occult blood in saliva. It is particularly desirable to provide a rapid test for gum bleeding so as to allow consumers to track the progress of treatments for disorders such as gingivitis.

Further areas of applicability of the present invention will become apparent from the detailed description provided hereinafter.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

As used herein, the term "cumene hydroperoxide" refers to 2-hydroperoxypropan-2-ylbenzene, as depicted below:

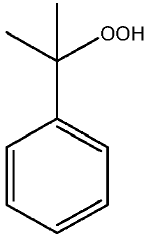

The use of 3,3',5,5'-tetramethylbenzidine (TMB) in combination with hydrogen peroxide as a presumptive test for blood has been reported (Garner et al., 1976. "An evaluation of tetramethylbenzidine as a presumptive test for blood", J Forensic Sci, 21, 4, pp 816-821). Proteins present in blood, particularly haemoglobin, display peroxidase activity. TMB acts as a hydrogen donor for the reduction of hydroperoxides by these proteins. The TMB is oxidized to 3,3',5,5'-tetramethylbenzidine diimine. This oxidation produces a colour change.

The use of TMB in combination with cumene hydroperoxide has also been proposed. Cumene hydroperoxide is reported to show good specificity for haemoglobin (EP0253548).

It has surprisingly been found that the inclusion of an anionic or non-ionic surfactant in an aqueous solution containing TMB and cumene hydroperoxide increases the rate of colour change in the presence of blood. Without wishing to be bound by theory, it is believed that the anionic or non-ionic surfactant solubilizes the cumene hydroperoxide thereby allowing the reaction to proceed more rapidly.

The composition is in the form of an aqueous solution. This allows good contact between the reagents and the sample. Additionally, the reagents are distributed throughout the solution, allowing the colour change to be observed easily. The use of a solution further allows quantitative detection of blood to be easily achieved, for example through the use of spectroscopic detection.

The concentration of TMB present in the solution is suitably selected such that the TMB is fully dissolved in the composition. In other words, the composition may be free of undissolved TMB. In the arrangements where TMB is to be used as a qualitative indicator, improved sensitivity is obtained when the TMB is dilute because the colour change may be observed more easily. In the arrangements where the composition is to be used with spectroscopic detection, the concentration may be selected such that the absorbance of the solution at 450 nm after complete oxidation of the TMB is in the range 0.1 to 1.2, preferably 0.3 to 1.0. For example, TMB may be present in the composition in an amount in the range of 1 % to 10 % by weight of the composition, optionally 4 % to 6 % by weight of the composition.

The amount of cumene hydroperoxide present in the composition is selected such that the cumene hydroperoxide is fully dissolved in the composition. The amount may vary depending on the nature and the amount of surfactant present in the composition. Illustratively, the cumene hydroperoxide may be present in an amount in the range of 0.1 % to 1 %, optionally 0.4 % to 0.6 %.

The surfactant may be an anionic surfactant or a non-ionic surfactant. Cationic surfactants are believed to solubilize cumene hydroperoxide weakly. Moreover, cationic surfactants have been reported to catalyse the decomposition of cumene hydroperoxide (Trunova et al, 2007. "Decomposition of cumene hydroperoxide in the systems of normal and reverse micelles formed by cationic surfactants", Colloid Journal, 69, no. 5, pp 655-659). The composition is therefore preferably substantially free of cationic surfactants. In this context, "substantially free" means that the amount of cationic surfactant is not sufficient to produce a colour change in the absence of blood. Trace amounts of cationic surfactants, for example no more than 0.05 %, no more than 0.025 %, no more than 0.01 % or preferably no more than 0.005 % cationic surfactant by weight of the composition, may be present.

The surfactant is most preferably an anionic surfactant. The nature of the anionic surfactant is not particularly limited. Examples of anionic surfactants useful herein include fatty acid monoglyceride monosulfates, alkyl sulfates (e.g., sodium lauryl sulfate); alkyl aryl sulfonates, (e.g., sodium linear dodecyl benzene sulfonate), olefin sulfonates (e.g., sodium olefin sulfonate), alkyl alkali sulfoacetates (e.g., sodium lauryl sulfoacetate); fatty acid esters of 1,2-dihydroxypropane sulfonates; the substantially saturated higher aliphatic acyl amides of lower aliphatic aminocarboxylic acid alkali metal salts (e.g, having 12 to 16 carbon atoms in the fatty acyl radicals), higher alkyl poly-lower alkoxy (of 10 to 100 alkoxies) sodium sulfates, and higher fatty acid sodium and potassium soaps of coconut oil and tallow.

The preferred classes of anionic surfactants are the alkyl sulfate surfactants and alkyl ether sulfate surfactants.

Alkyl sulfate surfactants are surfactants typically having general formula 1:

Formula 1: R¹-SO₃⁻M⁺

wherein R1 is an alkyl group and M⁺ is a counterion. The alkyl group may comprise a total of 8 to 50 carbon atoms, optionally 10 to 22 carbon atoms. The alkyl group may be linear or branched, and is preferably linear. The nature of the counterion is not particularly limited. Suitable counterions include sodium, potassium and ammonium.

The counterion is preferably ammonium. The alkyl group is preferably a linear alkyl group having 10 to 14 carbon atoms, more preferably 12 carbon atoms. The most preferred anionic surfactant is ammonium lauryl sulfate. Of the surfactants tested, ammonium lauryl sulfate was found to give the largest increase in reaction rate.

Alkyl ether sulfate surfactants are surfactants typically having general formula II:

Formula II: R²-(O-(CH₂)ₓ)_{y}-SO₃⁻M⁺

wherein R2 is an alkyl group, X is an integer from 1 to 5, Y is an integer from 1 to 10, and M is a counterion. The alkyl group may comprise a total of 8 to 50 carbon atoms, optionally 10 to 22 carbon atoms. The alkyl group may be linear or branched, and is preferably linear. Preferably, X is 2. Preferably, Y is from 2 to 5. The nature of the counterion is not particularly limited. Suitable counterions include sodium, potassium and ammonium.

R2 is preferably linear and preferably has 10 to 14 carbon atoms, more preferably 12 carbon atoms. The counterion is particularly preferably sodium. Most preferably, the alkyl ether sulfate is sodium lauryl ether sulfate.

In an alternative arrangement, the surfactant is a non-ionic surfactant. The nature of the non-ionic surfactant is not particularly limited. Examples of non-ionic surfactants useful in the compositions described herein include compounds produced by the condensation of alkylene oxide groups (hydrophilic in nature) with an organic hydrophobic compound which may be aliphatic or alkyl-aromatic in nature. Examples of suitable nonionic surfactants include poloxamers (sold under trade name Pluronic), polyoxyethylene sorbitan esters (sold under trade name Tweens), fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials.

The preferred non-ionic surfactants are the fatty acid amides of formula III: 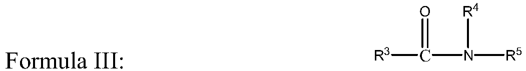 wherein R³ is a C8 to C50 linear or branched alkyl group; R⁴ is selected from hydrogen, C1 to C4 linear or branched alkyl, 2-hydroxy ethyl and 2-hydroxy propyl; and R⁵ is selected from 2-hydroxy ethyl and 2-hydroxy propyl. Preferably, R³ is a C9 to C19 linear alkyl group and R⁴ is hydrogen.

A preferred mixture of fatty acid amides is that mixture known in the art as coconut mono ethanol amide. Coconut mono ethanol amide is also referred to as cocamide mono ethanol amide. Coconut mono ethanol amide is obtainable by reacting ethanolamide with fatty acids derived from coconut oil. An example component of coconut mono ethanol amide is lauramide mono ethanol amide.

A mixture of surfactants may be present in the composition. The mixture may comprise two or more anionic surfactants, or two or more non-ionic surfactants, or at least one anionic surfactant and at least one non-ionic surfactant.

The amount of surfactant present in the composition is advantageously selected such that the surfactant solubilizes the cumene hydroperoxide. The amount of surfactant may vary depending on the amount of cumene hydroperoxide and the nature of the surfactant. For example, the surfactant may be present in an amount in the range of 0.5 % to 5 % by weight of the composition.

The composition may comprise one or more additional components, such as a pH adjusting agent. pH adjusting agents include a wide variety of common buffers, acidulants, and/or alkalizers which are well known to those skilled in the art. Examples include organic acids and their alkali metal salts, such as citric acid, malic acid, butyric acid, gluconic acid, adipic acid, glutaric acid, and malonic acid; amines such as triethanolamine and tris(hydroxyaminomethane); alkali metal hydroxides, such as sodium hydroxide, potassium hydroxide, and ammonium hydroxide; and combinations thereof. The optimum concentration of the pH-adjusting agent is the lowest level necessary to achieve the desired pH.

The composition is an aqueous solution and further comprises water.

In specific arrangements, the composition may be any of the detecting solutions identified in the Examples excluding the comparative solutions. The amounts of any of the components of these solutions may be varied by up to ±10 % or up to ±15 %.

Also provided herein is a kit comprising the composition for detecting blood as discussed above and an oral care composition.

The composition for detecting blood may be packaged in any suitable manner. The composition may be packaged in a plurality of containers, for example sachets or ampoules, each containing an amount of composition selected to allow the analysis of a single saliva sample. Packaging the composition in single-use containers in this manner allows for the more convenient use of the kit.

Alternatively, the kit may include a container containing an amount of composition sufficient to allow the analysis of a plurality of samples. This may reduce production costs. In this arrangement, the kit preferably includes a means for measuring a volume of the composition. The means may be, for example, a graduated vessel, a measuring spoon, a measuring cup, a syringe, or the like.

One of skill in the art will be familiar with the formulation of oral care compositions. Oral care compositions generally include at least an oral care active and an orally-acceptable carrier. Illustrative examples of ingredients which may be included in an oral care composition include abrasives, polymers, enzymes, humectants, thickeners, viscosity modifiers, antimicrobial agents, chelating agents, pH adjusting agents, preservatives, flavorings, sweeteners, whitening agents, colorants, herbal extracts and combinations thereof.

The oral care composition may be in any appropriate form. For example, the oral care composition may be a toothpaste, a tooth gel, a tooth powder, or a mouth rinse. The kit may include two or more oral care compositions, for example a toothpaste and a mouth rinse or a tooth gel and a mouth rinse.

The oral care composition is most preferably an oral care composition for use in treating gum bleeding or a disease or disorder associated with gum bleeding. The disease may be gingivitis. Suitable compositions are known in the art. Examples of useful compositions include those described in WO13/081627, WO13/081626, WO06/065522 and WO09/099452.

The kit may include a toothbrush. One of skill in the art would be familiar with the construction of toothbrushes. The nature of the toothbrush is not particularly limited. For example, the kit may comprise a plurality of disposable toothbrushes, such as those described in WO04/021914 or WO09/094231. Disposable toothbrushes are particularly advantageous in the arrangement where the toothbrush is to be contacted directly with a composition for detecting blood. Preferably, the toothbrush is adapted for use in the treatment or prevention of gingivitis. Useful toothbrushes include those described in US3,722,020.

The kit may include chewing gum. Chewing gum has been reported to be effective in treating or preventing gingivitis when used in combination with an oral care composition (see US 5340566). Other components which may be present in the kit include means for cleaning interproximal spaces, such as dental floss, interdental brushes, and the like.

Still further useful components may include a timer or stopwatch and means for terminating the reaction between cumene hydroperoxide and haemoglobin, for example an aqueous solution comprising 5 % to 15 % sulfuric acid by weight.

The kit may include printed matter, for example instructions regarding the use of the kit and/or a prescribed treatment regimen using the oral care composition. Usefully, the printed matter may include a calibrated colour scale for estimating the severity of bleeding.

An alternative kit includes the oral care composition and components for forming a composition as described herein. The required components are 3,3',5,5'-tetramethylbenzidine, cumene hydroperoxide, and a surfactant. The aqueous solvent may be included in the kit or supplied separately. If the aqueous solvent is included in the kit, then the surfactant may be provided as a solution in the aqueous solvent. The 3,3',5,5'-tetramethylbenzidine may be provided in a separate container to the cumene hydroperoxide.

The kits described herein are useful for allowing a consumer to assess the effect of the oral care composition on gum bleeding. In use, the consumer would use the oral care composition in the conventional manner. The consumer would expectorate a saliva sample and contact the saliva sample with the composition for detecting blood. If blood is present in the sample then a colour change will be observed. The extent of the colour change allows the extent of the bleeding to be estimated. It is desirable to allow consumers to associate an improvement in gum bleeding with a specific oral care composition.

Further provided herein is a method for detecting blood in a sample using the composition for detecting blood.

Blood includes haemoglobin, which acts as a peroxidase. The method includes the step of contacting the sample with a composition as defined above, such that oxidation of the 3,3',5,5'-tetramethylbenzidine by the cumene hydroperoxide is catalysed by the haemoglobin. The composition is optionally prepared using a kit as defined herein.

The nature of the contacting step may be selected as appropriate depending on the type of sample to be used. If the sample consists of a liquid, this step comprises mixing the sample with the composition. If the sample is carried on a solid object, for example a toothbrush, this step may comprise dipping the object in the composition. Spraying the composition onto an object is also contemplated.

The use of the method for forensic purposes is contemplated herein. In this arrangement the sample may be located on a solid object, the nature of which being not particularly limited. More preferably, the method is used in diagnosis. In this arrangement, the sample comprises a body fluid obtained from a patient. The method has been found to be particularly useful in the detection of occult blood in saliva and accordingly the sample most preferably comprises saliva.

The methods described herein are generally *in vitro* methods practised on existing samples. The methods do not necessarily include the step of obtaining a sample from a patient.

The method may be used to detect blood residue on a toothbrush after use. In this arrangement, the method is useful in the diagnosis of diseases and disorders of the oral cavity such as gingivitis.

The method also includes detecting the oxidation of the 3,3',5,5'-tetramethylbenzidine. The occurrence of oxidation indicates that blood is present. Oxidation of 3,3',5,5'-tetramethylbenzidine to 3,3',5,5'-tetramethylbenzidine diimine results in a colour change. Accordingly, the detection may comprise visual observation of the composition. Alternatively, oxidation may be detected spectroscopically.

The detection may be qualitative, semi-quantitative, or quantitative. In the arrangements where the detection is qualitative, the colour change associated with the oxidation of 3,3',5,5'-tetramethylbenzidine is used to identify the absence or presence of blood, generally by simple visual inspection.

In the arrangement where the detection is semi-quantitative, the colour of the composition after exposure to blood is compared with, for example, a calibrated colour scale to allow an estimate of the amount of blood present to be obtained. Quantitative detection generally involves the use of detection means, such as UV/visible spectrometer or colorimeter to measure the concentration of tetramethylbenzidine or 3,3',5,5'-tetramethylbenzidine diimide present in the composition.

Generally, measurements are taken at fixed time points, for example at 30 seconds, 60 seconds or 90 seconds after contacting the composition with the sample. Alternatively, the reaction may be allowed to proceed to completion before the measurement is taken.

Also described herein is the use of a surfactant to reduce the time taken to detect blood using 3,3',5,5'-tetramethylbenzidine and cumene hydroperoxide in an aqueous solution, wherein the surfactant is an anionic surfactant or a non-ionic surfactant. The reduction is relative to a comparative reaction mixture which does not include the anionic or non-ionic surfactant. The nature of the anionic surfactant or non-ionic surfactant is not particularly limited. The preferred surfactants are identified in the discussion of the compositions of the invention, above. Typically, the non-ionic surfactant is an alkyl sulfate surfactant or an alkyl ether sulfate surfactant, which has been found to be particularly effective in increasing the rate of reaction. Alternatively, the surfactant may be a non-ionic surfactant which is a fatty acid amide. Fatty acid amides have been shown to be effective in increasing the rate of reaction.

The blood to be detected may be occult blood in saliva. The compositions and methods described herein are particularly useful in the analysis of such samples.

### EXAMPLES

The present invention will now be explained by reference to the following illustrative examples.

### Example 1: Effect of a surfactant on reaction rate

The rate of oxidation of TMB in various compositions in the presence of blood was investigated. The inclusion of the alkyl sulfate surfactant ammonium lauryl sulfate in the composition was found to produce a significant increase in reaction rate.

A series of detecting solutions was prepared in accordance with Table 1. To three 20 mL aliquots of each solution there was added 100 µL of saliva containing blood. The reaction in each aliquot was terminated by adding 1 mL of 10 % sulfuric acid and after 10, 20 or 30 seconds at 450 nm was then measured using a spectrometer. The results of this investigation are set out in Table 1.

**Table 1: formulations of detecting solutions and optical densities at 450 nm at various time points.**

| Sample | Components of detecting solution / wt% | | | | | Reaction time /s | OD₄₅₀ |
|---|---|---|---|---|---|---|---|
| | Water | TMB | H₂O₂ | CHP | ALS | | |
| 1 (comparative) | 94.5 | 5.0 | 0.5 | - | - | 10 | 0.08 |
| 2 (comparative) | | | | | | 20 | 0.19 |
| 3 (comparative) | | | | | | 30 | 0.54 |
| 4 (comparative) | 94.5 | 5.0 | - | 0.5 | - | 10 | 0.09 |
| 5 (comparative) | | | | | | 20 | 0.21 |
| 6 (comparative) | | | | | | 30 | 0.49 |
| 7 | 92.5 | 5.0 | - | 0.5 | 2.0 | 10 | 0.14 |
| 8 | | | | | | 20 | 0.35 |
| 9 | | | | | | 30 | 0.61 |

As shown in Table 1, similar rates of reaction were observed in the solutions containing hydrogen peroxide (samples 1 to 3 (comparative)) and cumene hydroperoxide (CHP) in the absence of a surfactant (samples 4 to 6 (comparative)). The inclusion of ammonium lauryl sulfate in the solution was found to increase the rate of reaction. The absorbance of the composition of the invention at 450 nm after 20 seconds (sample 8) was 66.7 % greater than the comparative composition containing no surfactant (sample 5). Without wishing to be bound by theory, it is believed that the surfactant improves the mixing of the hydrophobic cumene hydroperoxide with the water, thereby improving reaction efficacy.

### Example 2: comparison of surfactants

The effects of alternative surfactants on reaction rate were investigated. Sodium lauryl ether sulfate (SLES) and coconut monoethanol amide (CMEA) were shown to be effective in increasing reaction rate.

A series of detecting solutions was prepared in accordance with Table 2. To 20 mL of each solution was added 100 µL of saliva containing blood. After 20 seconds, the reaction was terminated using 1 mL of 10 % sulfuric acid and the optical density of the solution recorded. The results are shown in Table 2.

**Table 2: detecting solutions containing various surfactants.**

| Sample | Components of detecting solution / wt% | | | | | | Reaction time /s | OD₄₅₀ |
|---|---|---|---|---|---|---|---|---|
| | Water | TMB | CHP | ALS | SLES | CMEA | | |
| 1 | 92.5 | 5.0 | 0.5 | 2.0 | - | - | 20 | 0.35 |
| 2 | 92.5 | 5.0 | 0.5 | - | 2.0 | - | 20 | 0.27 |
| 3 | 92.5 | 5.0 | 0.5 | - | - | 2.0 | 20 | 0.24 |
| 4 (comparative) | 92.5 | 5.0 | 0.5 | - | - | - | 20 | 0.21 |

As shown in the Table above, each of the surfactants tested produced an increase in reaction rate in comparison to sample 4 containing no surfactant. The greatest increase was observed for ammonium lauryl sulfate.

## Claims

1. A kit comprising:
a) a composition for detecting blood on a solid object, which composition includes:
(i) 3,3',5,5'-tetramethylbenzidine,
(ii) cumene hydroperoxide, and
(iii) a surfactant;
wherein the composition is an aqueous solution; and
wherein the surfactant is an anionic surfactant or a non-ionic surfactant, and
b) an oral care composition.

2. The kit of claim 1, wherein the solid object is a toothbrush.

3. The kit of claim 1, wherein the kit further comprises as a means for terminating the reaction between cumene hydroperoxide and haemoglobin contained in the blood an aqueous solution comprising 5% to 15% sulfuric acid by weight.

4. The kit of claim 1, wherein the surfactant is an anionic surfactant.

5. The kit of claim 4, wherein the anionic surfactant is an alkyl sulfate surfactant or an alkyl ether sulfate surfactant.

6. The kit of claim 5, wherein the alkyl sulfate surfactant is ammonium lauryl sulfate.

7. The kit of claim 5, wherein the alkyl ether sulfate surfactant is sodium lauryl ether sulfate.

8. The kit of claim 1, wherein the surfactant is a non-ionic surfactant.

9. The kit of claim 8, wherein the non-ionic surfactant is a fatty acid amide.

10. The kit of claim 9, wherein the fatty acid amide is coconut mono ethanol amide.

11. The kit of any preceding claim,
wherein the oral care composition is for use in the treatment of gingivitis.

12. A method for detecting blood in a sample, the blood including haemoglobin, which method comprises:
(a) contacting the sample with the composition as defined in any one of claims 1 to 11, wherein the oxidation of the 3,3',5,5'-tetramethylbenzidine by the cumene hydroperoxide is catalysed by the haemoglobin, and
(b) detecting the oxidation of the 3,3',5,5'-tetramethylbenzidine as indicating the presence of blood.

13. The method of claim 12, wherein the sample comprises saliva.

14. The method of claim 12 or claim 13, wherein the sample is carried on a toothbrush.

## Patentansprüche

1. Kit, umfassend:
a) eine Zusammensetzung zur Detektierung von Blut auf einem festen Objekt, wobei die Zusammensetzung einschließt:
(i) 3,3',5,5'-Tetramethylbenzidin,
(ii) Cumenhydroperoxid, und
(iii) ein Tensid;
wobei die Zusammensetzung eine wässrige Lösung ist, und
wobei das Tensid ein anionisches Tensid oder ein nichtionisches Tensid ist, und
b) eine Mundpflegezusammensetzung.

2. Kit nach Anspruch 1, wobei das feste Objekt eine Zahnbürste ist.

3. Kit nach Anspruch 1, wobei das Kit weiterhin umfasst, als ein Mittel zur Beendigung der Reaktion zwischen Cumenhydroperoxid und Hämoglobin, das in dem Blut enthalten ist, eine wässrige Lösung, die 5 Gew.-% bis 15 Gew.-% Schwefelsäure umfasst.

4. Kit nach Anspruch 1, wobei das Tensid ein anionisches Tensid ist.

5. Kit nach Anspruch 4, wobei das anionische Tensid ein Alkylsulfat-Tensid oder eine Alkylethersulfat-Tensid ist.

6. Kit nach Anspruch 5, wobei das Alkylsulfat-Tensid Ammoniumlaurylsulfat ist.

7. Kit nach Anspruch 1, wobei das Alkylethersulfat-Tensid Natriumlaurylethersulfat ist.

8. Kit nach Anspruch 1, wobei das Tensid ein nichtionisches Tensid ist.

9. Kit nach Anspruch 8, wobei das nichtionische Tensid Fettsäureamid ist.

10. Kit nach Anspruch 9, wobei das Fettsäureamid Kokosnuss-Monoethanolamid ist.

11. Kit nach irgendeinem vorhergehenden Anspruch, wobei die Mundpflegezusammensetzung für die Verwendung in der Behandlung von Gingivitis ist.

12. Verfahren zur Detektierung von Blut in einer Probe, wobei das Blut Hämoglobin einschließt, wobei das Verfahren umfasst:
(a) In-Kontaktbringen der Probe mit der Zusammensetzung wie in irgendeinem der Ansprüche 1 bis 11 definiert, wobei die Oxidation von 3,3',5,5'-Tetramethylbenzidin durch Cumenhydroperoxid durch das Hämoglobin katalysiert wird, und
(b) Detektierung der Oxidation von 3,3',5,5'-Tetramethylbenzidin als Anzeige der Anwesenheit von Blut.

13. Verfahren nach Anspruch 12, wobei die Probe Speichel umfasst.

14. Verfahren nach Anspruch 12 oder Anspruch 13, wobei die Probe auf einer Zahnbürste getragen wird.

## Revendications

1. Kit comprenant:
a) une composition pour la détection de sang sur un objet solide, laquelle composition comprend :
(i) de la 3,3',5,5'-tétraméthylbenzidine;
(ii) de l'hydroperoxyde de cumène; et
(iii) un tensio-actif;
dans lequel la composition est une solution aqueuse ; et
dans lequel le tensio-actif est un tensio-actif anionique ou un tensio-actif non ionique; et
b) une composition de soin buccal.

2. Kit selon la revendication 1, dans lequel l'objet solide est une brosse à dents.

3. Kit selon la revendication 1, dans lequel le kit comprend en outre, en tant que moyen pour terminer la réaction entre l'hydroperoxyde de cumène et l'hémoglobine contenue dans le sang, une solution aqueuse comprenant 5 % à 15 % d'acide sulfurique en poids.

4. Kit selon la revendication 1, dans lequel le tensio-actif est un tensio-actif anionique.

5. Kit selon la revendication 4, dans lequel le tensio-actif anionique est un tensio-actif alkyl sulfate ou un tensio-actif alkyl éther sulfate.

6. Kit selon la revendication 5, dans lequel le tensio-actif alkyl sulfate est le lauryl sulfate d'ammonium.

7. Kit selon la revendication 5, dans lequel le tensio-actif alkyl éther sulfate est le lauryl éther sulfate de sodium.

8. Kit selon la revendication 1, dans lequel le tensio-actif est un tensio-actif non ionique.

9. Kit selon la revendication 8, dans lequel le tensio-actif non ionique est un amide d'acide gras.

10. Kit selon la revendication 9, dans lequel l'amide d'acide gras est le mono éthanol amide de noix de coco.

11. Kit selon l'une quelconque des revendications précédentes,
dans lequel la composition de soin buccal est pour une utilisation dans le traitement de la gingivite.

12. Procédé pour la détection de sang dans un échantillon, le sang comprenant de l'hémoglobine, lequel procédé comprend:
(a) mettre en contact l'échantillon avec la composition telle que définie dans l'une quelconque des revendications 1 à 11, l'oxydation de la 3,3',5,5'-tétraméthylbenzidine par l'hydroperoxyde de cumène étant catalysée par l'hémoglobine; et
(b) détecter l'oxydation de la 3,3',5,5'-tétraméthylbenzidine comme indiquant la présence de sang.

13. Procédé selon la revendication 12, dans lequel l'échantillon comprend de la salive.

14. Procédé selon l'une des revendications 12 ou 13, dans lequel l'échantillon est porté sur une brosse à dents.
